(19) Europäisches Patentamt
European Patent Office
Office européen des brevets

(11) Veröffentlichungsnummer: **0 463 139 B1**

(12) # EUROPÄISCHE PATENTSCHRIFT

(45) Veröffentlichungstag der Patentschrift: **12.07.95**

(51) Int. Cl.[6]: **C07D 457/12**, A61K 31/48

(21) Anmeldenummer: **91902179.0**

(22) Anmeldetag: **15.01.91**

(86) Internationale Anmeldenummer:
**PCT/DE91/00039**

(87) Internationale Veröffentlichungsnummer:
**WO 91/10663 (25.07.91 91/17)**

Die Akte enthält technische Angaben, die nach dem Eingang der Anmeldung eingereicht wurden und die nicht in dieser Patentschrift enthalten sind.

(54) **2,13-DISUBSTITUIERTE ERGOLINE, DEREN HERSTELLUNG UND VERWENDUNG IN ARZNEIMITTELN.**

(30) Priorität: **15.01.90 DE 4001323**

(43) Veröffentlichungstag der Anmeldung:
**02.01.92 Patentblatt 92/01**

(45) Bekanntmachung des Hinweises auf die Patenterteilung:
**12.07.95 Patentblatt 95/28**

(84) Benannte Vertragsstaaten:
**AT BE CH DE DK ES FR GB GR IT LI LU NL SE**

(56) Entgegenhaltungen:
**EP-A- 0 160 842**
**EP-A- 0 217 734**
**EP-A- 0 220 129**
**WO-A-89/08109**

**Chemical Abstracts, Band 112, 1990, Columbus, Ohio, US, Seite 764, Zusammenfassung-Nr. 198878q & CS, A, 262283**

(73) Patentinhaber: **SCHERING AKTIENGESELLSCHAFT**

**D-13342 Berlin (DE)**

(72) Erfinder: **SAUER, Gerhard, Dr.**
**Königsbacher Zeile 41a**
**D-1000 Berlin 28 (DE)**
Erfinder: **SCHRÖTER, Bernd**
**Winterstr. 6**
**D-1000 Berlin 51 (DE)**
Erfinder: **BRUMBY, Thomas, Dr.**
**Hauptstr. 13**
**D-1000 Berlin 62 (DE)**
Erfinder: **WACHTEL, Helmut, Dr.**
**Suarezstr. 22**
**D-1000 Berlin 19 (DE)**
Erfinder: **LÖSCHMANN, Peter-Andreas**
**Württembergallee 8**
**D-1000 Berlin 19 (DE)**

**EP 0 463 139 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

Chemical Abstracts, Band 112, 1990, Columbus, Ohio, US, Seite 826, Zusammenfassung-Nr. 179583y & CS, A, 262282

## Beschreibung

Die Erfindung betrifft neue 2,13-disubstituierte Ergoline, deren Herstellung und Verwendung in Arzneimitteln, sowie Zwischenprodukte zur Herstellung derselben.

Aus EP-A-220 129 sind 13-substituierte Ergoline bekannt, die Affinität zu zentralen Dopamin-Rezeptoren aufweisen. Die neuen 2,13-disubstituierten Ergoline zeigen eine höhere Affinität zum Dopamin-Rezeptor bei verbesserter metabolischer Stabilität und somit eine Wirkungssteigerung.

Die Erfindung betrifft Verbindungen der Formel I und deren Säureadditonssalze

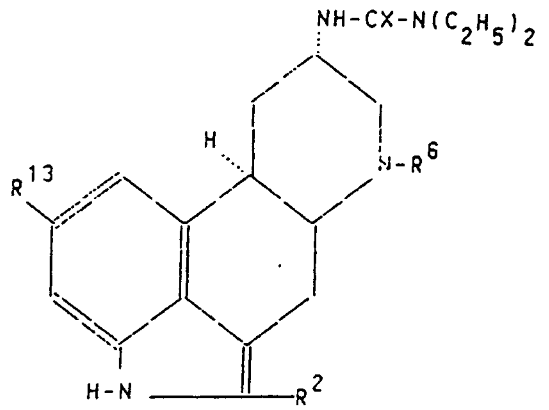

I

worin

- $R^2$ Halogen, $C_{1-6}$-Alkyl oder -S-$C_{1-4}$-Alkyl,
- $R^6$ $C_{1-6}$-Alkyl, $C_{3-6}$-Alkenyl oder $C_{3-5}$-Cycloalkyl-$C_{1-2}$-alkyl,
- X Sauerstoff oder Schwefel,
- $R^{13}$ Chlor, Jod, -S-$C_{1-4}$-Alkyl, $C_{1-6}$-Alkyl, $C_{2-6}$-Alkenyl, 1,3-Dithiolan-2-yl, -CO-H oder -$CR^4R^5$OH ist und $R^4$ und $R^5$ jeweils Wasserstoff oder $C_{1-5}$-Alkyl bedeuten.

Die physiologisch verträglichen Säureadditionssalze leiten sich von den bekannten anorganischen und organischen Säuren ab wie zum Beispiel Salzsäure, Schwefelsäure, Bromwasserstoffsäure, Zitronensäure, Maleinsaure, Fumarsäure, Weinsäure u.a..

Halogen beinhaltet insbesondere Chlor, Brom und Jod.

Unter Alkyl ist jeweils ein geradkettiger oder verzweigter Alkylrest zu verstehen wie beispielsweise Methyl, Ethyl, n-Propyl, Isopropyl, n-Butyl, Isobutyl, sek. Butyl, tert. Butyl, Pentyl, Hexyl, 2,2-Dimethylpropyl, 2-Methylbutyl, Isopentyl u.a..

Bedeuten $R^6$ oder $R^{13}$ einen Alkenylrest, so kann dieser geradkettig oder verzweigt sein und enthält bevorzugt nur eine Doppelbindung, wobei die Doppelbindung in $R^6$ nicht benachbart zum Stickstoffatom stehen kann. Als Alkenylreste sind beispielsweise geeignet: Vinyl, 1-Propenyl, 2-Propenyl, 1-Methyl-2-propenyl, 1-Butenyl, Methallyl.

Bedeutet $R^6$ eine Cyclo-alkyl-alkyl-Gruppe, so sind Reste mit bis zu 5 Kohlenstoffatomen, beispielsweise Cyclopropylmethyl, Cyclopropylethyl und Cyclobutylmethyl bevorzugt.

Bedeuten $R^2$, $R^6$ und $R^{13}$ Alkyl- oder Alkenylreste, so sind solche mit bis zu 4 Kohlenstoffatomen als bevorzugt zu betrachten.

Die Verbindungen der Formel I können als E- oder Z-Isomere oder, falls ein chirales Zentrum im Rest $R^2$ oder $R^{13}$ vorhanden ist, als Diastereomere und als Gemische derselben auftreten. Die Isomeren und Isomerengemische sind von der vorliegenden Erfindung auch umfaßt.

Die Verbindungen der Formel I sowie deren Säureadditionssalze sind auf Grund ihrer Affinität zu zentralen Dopaminrezeptoren als Arzneimittel verwendbar. Je nach Art der Substituenten in 2-, 13- und 6-Stellung sind sie Dopaminagonistisch, -antagonistisch oder partialagonistisch wirksam und eignen sich beispielsweise zur Behandlung des Morbus Parkinson, der Hyperprolaktinämie, der positiven oder negativen Symptomatik bei Schizophrenie oder der Emesis.

Die dopaminagonistische Wirkung wird beispielsweise mit Hilfe der von Horowski beschriebenen Methode der automatischen Registrierung von Stereotypien an Ratten bestimmt (Arzneim. Forsch. 12, 2281-2286, 1978): Unmittelbar nach intraperitonealer Prüfsubstanz- bzw. Vehikelverabreichung werden männliche Wistar-Ratten (90-120 g) einzeln in Zwangskäfige aus Acrylglas gesetzt. Uber ein vor dem Kopf der Tiere

angebrachtes elektrodynamisches Aufnahmesystem wird die Zahl der Kontakte an einem stählernen Becher mit einem zentralen Metallstab als Folge der stereotypen Kau-, Leck- und Nagebewegungen während 60 Minuten registriert. Die Mittelwerte ± S.E.M. der Anzahl der Kontakte während 60 Minuten für die verschiedenen Behandlungsgruppen, die mit jeweils 12 Tieren besetzt sind, werden berechnet und die Signifikanz der Unterschiede zwischen den Mittelwerten der verschiedenen Prufsubstanzdosen im Vergleich zur Vehikelbehandelten Kontrollgruppe mit Hilfe der einfachen Varianzanalyse in Verbindung mit dem Dunnett-Test ermittelt. Die Ergebnisse sind in der Tabelle 1 dargelegt.

**TABELLE 1**

**Auslösung von Stereotypien bei Ratten während 60 Minuten nach intraperitonealer Behandlung mit Vehikel bzw. verschiedenen Dosen von Ergolinharnstoff-Derivaten**

(x: $p < 0.05$, xx: $p < 0.01$, Varianzanalyse/Dunnett-Test vs. Kontrolle; n: Anzahl der Tiere)

| | | | Stereotypien (Counts pro 60 Minuten) Mittelwert ± S.E.M.) Prüfsubstanzdosis (mg/kg) | | | | | |
|---|---|---|---|---|---|---|---|---|
| | n | Kontrolle | 0.025 | 0.1 | 0.39 | 1.56 | 6.25 |
| A | 12 | 624 ± 101 | 617 ± 124 | 2582 ± 517 | 5834 ± 951xx | 7563 ± 804xx | 6948 ± 1090xx |
| B | 12 | 928 ± 120 | 1373 ± 448 | 1896 ± 270 | 5060 ± 607xx | 6929 ± 872xx | 4720 ± 970xx |
| C | 10 | 944 ± 84 | 1176 ± 183 | 1482 ± 170 | 5674 ± 662xx | 6594 ± 1128xx | 7111 ± 711xx |

A = 1,1-Diethyl-3-(2,13-dimethyl-6-propyl-8α-ergolinyl)-harnstoff

B = 1,1-Diethyl-3-(13-ethyl-2-methyl-6-propyl-8α-ergolinyl)-harnstoff

C = 8α-(3,3-Diethylureido)-2-methyl-6-propyl-ergolin-13-carbaldehyd

Da sich die erfindungsgemäßen Verbindungen insbesondere durch dopaminagonistische Wirkung auszeichnen, eignen sie sich insbesondere zur Behandlung des Morbus Parkinson.

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel

Wasser, Gelatine, Gummi arabicum, Milchzucker, Stärke, Magnesiumstearat, Talk, pflanzliche Ole, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüber hinaus Hilfsstoffe wie Konservierungs-, Stabilisierungs-, Netzmittel oder Emulgatoren, Salze zur Veränderung des osmotischen Drucks oder Puffer.

Die erfindungsgemäßen Verbindungen werden in einer Dosis von 0,001 bis 10 mg aktiver Substanz in einen physiologisch verträglichen Träger eingebracht. Die Anwendung der erfindungsgemäßen Verbindungen erfolgt in einer Dosis von 0,00001 bis 0,1 mg/kg/Tag, vorzugsweise 0,001 bis 0,1 mg/kg/Tag analog dem bekannten Mittel Bromocryptin.

Die Herstellung der erfindungsgemäßen Verbindungen der Formel I kann nach an sich bekannten Methoden durchgeführt werden.

Beispielsweise gelangt man zu Verbindungen der Formel I, indem man

a) Verbindungen der Formel II

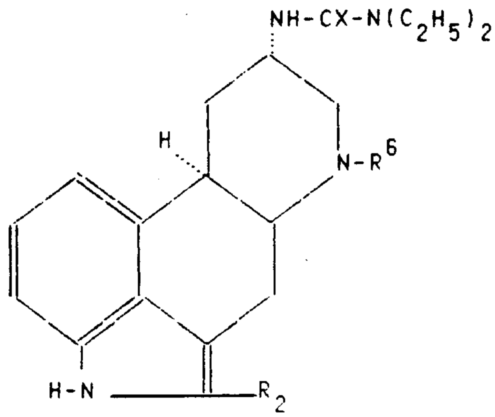

II

worin $R^2$, $R^6$ und X die obige Bedeutung haben, in Gegenwart einer Säure mit einem elektrophilen Agens umsetzt oder

b) Verbindungen der Formel III

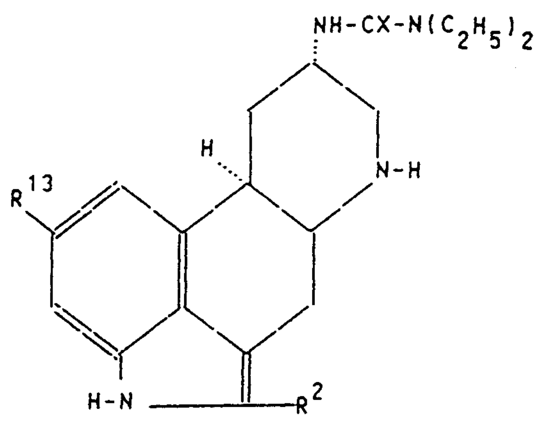

III

worin $R^2$, $R^{13}$ und X die obige Bedeutung haben, alkyliert oder alkenyliert zu Verbindungen mit $R^6$ in der obigen Bedeutung und gewünschtenfalls anschließend

α) Verbindungen mit $R^{13}$ = -CO-$R^3$ mit $R^3$=H oder $C_{1-5}$-Alkylreduziert zu Verbindungen mit $R^{13}$ = -$CR^4R^5OH$ und diese gewünschtenfalls dehydratisiert zu Verbindungen mit $R^{13}$ = $C_{2-6}$-Alkenyl oder reduziert zu Verbindungen mit $R^{13}$ = $C_{1-6}$-Alkyl oder

β) Verbindungen mit $R^{13}$ = 1,3-Dithiolan-2-yl überführt in Verbindungen mit $R^{13}$ = -CHO oder $CH_3$ oder

γ) den Harnstoff in den Thioharnstoff überführt oder

δ) die Isomeren trennt oder die Säureadditionssalze bildet.

Die elektrophile Substitution in 13-Stellung nach Verfahren a) wird in Gegenwart einer Säure bei Temperaturen von 0 °C bis 20 °C durchgeführt und ist im allgemeinen nach 1 bis 24 Stunden beendet.

Als Säuren können anorganische Säuren wie Phosphorsäure, Schwefelsäure, organische Säuren wie Trifluoressigsäure, Methansulfonsäure, Essigsäure - und LewisSäuren wie Aluminiumchlorid, Titanchlorid, Dimethylaluminiumchlorid, Zinntetrachlorid, Borfluorid u.ä. eingesetzt werden, wobei die organische Säure als Lösungsmittel dienen kann oder inerte aprotische Lösungsmittel zugesetzt werden wie chlorierte Kohlenwasserstoffe wie Dichlormethan, Chloroform., Tetrachlorethan oder Nitrobenzol.

Geeignete Elektrophile sind beispielsweise: Acylchloride wie Acetylchlorid, Propionylchlorid; Halogenierungsmittel wie N-Chlor-Succinimid, N-Jod-Succinimid, Trichlorisocyanursäure; Dimethyl-methylthio-sulfoniumtetrafluorborat; Dichlormethylalkylether; Chlorameisensdurealkylester; Ameisensäurealkylester und Ethandithiol bzw. Dithiolan u.a.

Die Substitution in 6-Stellung nach dem Verfahren b) kann beispielsweise nach A. Cerny et al. Coll. Czech. Chem. Comm. 49, 2828 (1984) oder nach dem in der EP-21206 beschriebenen Verfahren durchgeführt werden, indem man die 6-H-Verbindung der Formel II mit den entsprechenden $R^6$-Halogeniden (Bromiden, Chloriden, Iodiden) umsetzt. Zweckmäßigerweise erfolgt die Reaktion in einem inerten Lösungsmittel wie Ethanol, Dimethylformamid, Acetonitril oder Nitromethan in Gegenwart von Basen wie DBU, Alkalihydroxiden oder -carbonaten.

Verbindungen der Formel I mit $R^{13}$ in der Bedeutung einer -$COR^3$-Gruppe können nach den üblichen Verfahren zum Alkohol reduziert werden wie beispielsweise mit Lithiumaluminiumhydrid oder Lithiumtri-tert.-butoxyalanat in einem aprotischen Lösungsmittel wie cyclischen oder acyclischen Ethern beispielsweise Tetrahydrofuran, Dioxan, Diethylether. Durch Grignardierung oder Lithium-alkylierung können auch 1-Hydroxy-alkylierte Substituenten $R^{13}$ dargestellt werden. Die Grignardierung kann mit den üblichen Grignard-Reagenzen wie Alkylmagnesium-halogeniden in einem aprotischen Lösungsmittel wie cyclischen und acyclischen Ethern bei Temperaturen von - 70 °C bis 20 °C erfolgen, Die Umsetzung mit Alkyl-Lithium erfolgt unter analogen Bedingungen.

Die anschließende Dehydratisierung zur Doppelbindung kann in üblicher Weise vorgenommen werden, wie beispielsweise mit Sulfonaten oder Acetaten in polaren Lösungsmitteln wie Ethern in Gegenwart einer Base und gegebenenfalls unter Erwärmen.

Die Reduktion der Alkohole zu 13-Alkyl-Derivaten kann beispielsweise durch Umsetzung mit $NaBH_4$ in Essigsäure oder durch Reduktion mit Lithium in Ammoniak erfolgen.

Zur Einführung der 13-$CH_3$-Gruppe kann es vorteilhaft sein, vor der Reduktion des 13-$CH_2$-OH-Restes diesen mit Säuren wie Pivalinsäure, Essigsaure, Benzoesäure zu verestern und anschließend nach den bekannten Verfahren wie in der Deutschen Patentanmeldung P 4020341.7 beschrieben zu reduzieren.

Bedeutet $R^{13}$ einen Dithiolan-Rest, so kann dieser beispielsweise durch wässrige $SiO_2$-Behandlung und nachfolgender Umsetzung mit Sulfurylchlorid in aprotischen Lösungsmitteln wie chlorierten Kohlenwasserstoffen in das 13-Formyl-Derivat überführt werden. Durch Umsetzung mit Raney-Nickel bei Raumtemperatur in protischen Lösungsmitteln wie Alkoholen kann das 13-Methyl-Derivat dargestellt werden.

Die Uberführung der Harnstoffderivate in die Thioharnstoffe kann beispielsweise nach dem in der EP-A-217730 beschriebenen Verfahren durch Umsetzung mit Phosphoroxychlorid und einem Thiolierungsmittel erfolgen.

Die Isomerengemische können nach den üblichen Methoden wie beispielsweise Kristallisation, Chromatographie oder Salzbildung in die Diastereomeren bzw. E/Z-Isomeren aufgetrennt werden.

Die Verbindungen der Formel I werden entweder als freie Basen oder in Form ihrer physiologisch verträglichen Säureadditonssalze isoliert.

Zur Bildung von Salzen wird eine Verbindung der Formel I beispielsweise in wenig Alkohol oder Methylenchlorid gelöst und mit einer konzentrierten Lösung der gewünschten Säure versetzt.

Die Einführung des Substituenten in 6-Stellung kann vor oder nach Substitution in 13-Stellung erfolgen. Die Erfindung umfaßt auch die Verbindungen der Formel IV, die wertvolle Zwischenprodukte zur Herstellung pharmakologisch wirksamer Verbindungen darstellen. Die Reduzierung des 6-Cyan-Ergolins zur 6-H-Verbindung erfolgt beispielsweise nach A. Cerny et al. Coll. Czech. Chem, Comm. 49, 2828 (1984). Die Umwandlung der Zwischenprodukte in die Wirksubstanz erfolgt nach dem bei der Verfahrensvariante b) beschriebenen Methode.

Soweit die Herstellung der Ausgangsverbindungen nicht beschrieben wird, sind diese bekannt oder analog zu bekannten Verbindungen oder hier beschriebenen Verfahren herstellbar.

Die nachfolgenden Beispiele sollen das erfindungsgemäße Verfahren erläutern.

**Beispiel 1**

1,1-Diethyl-3-[13-(1,3-dithiolan-2-yl)-2,6-dimethyl-8α-ergolinyl]-harnstoff

5,31 g 1,1-Diethyl-3-(2,6-dimethyl-8α-ergolinyl)-harnstoff (15 mmol) löst man in 150 ml Chloroform und 50 ml Ameisensäureethylester, gibt 2,8 ml Ethandithiol (33 mmol) und 60 ml einer 1 molaren Lösung von Titantetrachlorid in Dichlormethan (60 mmol) zu und rührt 20 Stunden bei Raumtemperatur. Dann versetzt man mit 40 ml Methanol und 300 ml Wasser, macht mit 30 ml 25%iger Ammoniaklösung alkalisch und schüttelt mit Dichlormethan aus. Die organischen Phasen werden mit Natriumsulfat getrocknet, eingedampft und der Rückstand an Kieselgel mit Dichlormethan/Methanol chromatographiert. Man isoliert 4,14 g Substanz, die aus Essigester kristallisiert, $[\alpha]_D$ = - 4 ° (0.5% in Chloroform).

In analoger Weise werden hergestellt:

Aus 1,1-Diethyl-3-(2-methyl-6-propyl-8α-ergolinyl)-harnstoff der 1,1-Diethyl-3-[13-(1,3-dithiolan-2-yl)-2-methyl-6-propyl-8α-ergolinyl]-harnstoff in 38 % Ausbeute.

Aus 1,1-Diethyl-3-(2-ethyl-6-propyl-8α-ergolinyl)-harnstoff der 1,1-Diethyl-3-[13-(1,3-dithiolan-2-yl)-2-ethyl-6-propyl-8α-ergolinyl]-harnstoff in 28 % Ausbeute.

**Beispiel 2**

8α-(3,3-Diethylureido)-2,6-dimethy-ergolin-13-carbaldehyd

3,26 g 1,1-Diethyl-3-[13-(1,3-dithiolan-2-yl)-2,6-dimethyl-8α-ergolinyl]-harnstoff (7,1 mmol) löst man in 35 ml Chloroform und versetzt mit 5,3 g Kieselgel und 5,7 ml Wasser. Dann tropft man innerhalb von 10 Minuten die Lösung von 1,37 ml Sulfurylchlorid (17 mmol) in 35 ml Chloroform zu und rührt 1 Stunde bei Raumtemperatur. Nach Zugabe von 8,5 g Kaliumcarbonat wird 15 Minuten gerührt, etwas Ethanol und gesättigte Kochsalzlösung zugegeben und mit Dichlormethan ausgeschüttelt. Die organischen Phasen werden getrocknet und eingedampft, der Rückstand an Kieselgel mit Essigester/ Methanol chromatographiert` Ausbeute 1,45 g. Diese Substanz wird aus Essigester/Diisopropylether kristallisiert, Ausbeute 1,09 g (40 % der Theorie), $[\alpha]_D$ = - 9 ° (0,5 % in Chloroform).

In analoger Weise werden dargestellt:

Aus 1,1-Diethyl-3-[13-(1,3-dithiolan-2-y-)-2-methyl-6-propyl-8α-ergolin yl)harnstoff der 8α-(3,3-Diethylureido)-2-methyl-6-propyl-ergolin-13-carbaldehyd, Ausbeute 51 %.

Aus 1,1-Diethyl-3-[13-(1,3-dithiolan-2-yl)-2-ethyl-6-propyl-8α-ergolinyl)harnstoff der 8α-(3,3-Diethylureido)-2-ethyl-6-propyl-ergolin-13-carbaldehyd in 62 % Ausbeute.

**Beispiel 3**

2-Brom-8α-(3,3-diethylureido)-6-methyl-ergolin-13-carbaldehyd

Man löst 838 mg 3-(2-Brom-6-methyl-8α-ergolinyl)-1,1-diethyl-harnstoff (2 mmol) in 100 ml Dichlormethan, gibt 1,2 g wasserfreies Aluminiumchlorid (9 mmol) und 1,8 ml Dichlormethylmethylether (20 mmol) zu und rührt 15 Minuten bei Raumtemperatur. Man versetzt die Reaktionsmischung mit Eis, nach 15 Minuten mit einer Lösung von 1,5 g Weinsäure in 50 ml Wasser und macht mit 5 ml konz. Ammoniaklösung alkalisch. Man extrahiert mit Dichlormethan, trocknet die organischen Phasen mit Natriumsulfat und destilliert das Lösungsmittel ab, Der Rückstand wird an Kieselgel mit Dichlormethan/Methanol chromatographiert, man isoliert 406 mg (45 % der Theorie).

In analoger Weise werden dargestellt:

Aus 3-(2-Brom-6-propyl-8α-ergolinyl)-1,1-diethyl-harnstoff den 2-Brom-8α-(3,3diethylureido)-6-propyl-ergolin-13-carbaldehyd, Ausbeute 27 %.

Aus 3-(2-Chlor-6-methyl-8α-ergolinyl)-1,1-diethyl-harnstoff den 2-Chlor-8α-(3,3-diethylureido)-6-methyl-ergolin-13-carbaldehyd in 37 % Ausbeute.

Aus 3-(2-Chlor-6-propyl-8α-ergolinyl)-1,1-diethyl-harnstoff den 2-Chlor-8α-(3,3-diethylureido)-6-propyl-ergolin-13-carbaldehyd in 32 % Ausbeute.

Aus 1,1-Diethyl-3-(2-methylthio-6-propyl-8α-ergolinyl)-harnstoff den 8α-(3,3-Diethylureido)-2-methylthio-6-proply-ergolin-13-carbaldehyd in 42 % Ausbeute.

**Beispiel 4**

1,1-Diethyl-3-(2,6-dimethyl-13-hydroxymethyl-8α-ergolinyl)-harnstoff

370 mg 8 α-(3,3-Diethylureido)-2,6-dimethyl-ergolin-13-carbaldehyd (1 mmol) werden in 50 ml Tetrahydrofuran gelöst und mit 200 mg Lithiumaluminiumhydrid bei Raumtemperatur 1 Stunde reduziert. Die Mischung wird im Eisbad abgekühlt und nacheinander mit 0,2 ml Wasser, 0,2 ml 15%iger Natronlauge und 0,6 ml Wasser versetzt, der Niederschlag abfiltriert und das Filtrat zur Trockene eingedampft. Der Rückstand wird an Kieselgel mit Dichlormethan/Methanol chromatographiert. Man isoliert 243 mg Alkohol, der aus Essigester kristalliert. Ausbeute 166 mg (44 % der Theorie), $[\alpha]_D$ = + 6° (0,5 % in Chloroform).

In analoger Weise werden aus den betreffenden Aldehyden dargestellt:
1,1-Diethyl-3-(13-hydroxymethyl-2-methyl-6-proplyl-8α-ergolinyl)-harnstoff, Ausbeute 73 %.
1,1-Diethyl-3-(2-ethyl-13-hydroxymethyl-6-propyl-8α-ergolinyl)-harnstoff, Ausbeute 43 %.
3-(2-Brom-13-hydroxymethyl-6-methyl-8α-ergolinyl)-1,1-diethyl-harnstoff, Ausbeute 51 %.
3-(2-Brom-13-hydroxymethyl-6-propyl-8α-ergolinyl)-1,1-diethyl-harnstoff, Ausbeute 62 %.
3-(2-Chlor-13-hydroxymethyl-6-propyl-8α-ergolinyl)-1,1-diethyl-harnstoff, Ausbeute 42 %.
1,1-Diethyl-3-(13-hydroxymethyl-2-methylthio-6-propyl-8α-ergolinyl)-harnstoff, Ausbeute 34 %.

**Beispiel 5**

1,1-Diethyl-3-(2,6,13-trimethyl-8α-ergolinyl)-harnstoff

458 mg 1,1-Diethyl-3-[13-(1,3-dithiolan-2-yl)-2,6-dimethyl-8α-ergolinyl)-harnstoff (1 mmol) werden in 50 ml Methanol gelöst und bei Raumtemperatur mit mehreren Portionen Raney-Nickel behandelt, bis das Ausgangsmaterial nach DC verschwunden ist. Man filtriert durch Kieselgur, dampft das Lösungsmittel ein und chromatographiert den Rückstand an Kieselgel mit Dichlormethan/Methanol, die isolierte Substanz wird aus Methanol kristallisiert, Ausbeute 117 mg (31 % der Theorie).

**Beispiel 6**

1,1-Diethyl-3-(2,6,13-trimethyl-8α-ergolinyl)-harnstoff

In 10 ml Pyridin löst man 715 mg 1,1-Diethyl-3-(2,6-dimethyl-13-hydroxymethyl8α-ergolinyl)-harnstoff (1,5 mmol) und versetzt mit 2 ml Trimethylacetylchlorid. Nach 30 Minuten Rühren bei Raumtemperatur gibt man Eis zu, rührt weitere 30 Minuten, macht mit Ammoniak alkalisch und kühlt die Mischung im Eisbad. Die ausgeschiedenen Kristalle werden abgesaugt, Ausbeute 785 mg, $[\alpha]_D$ = - 2 ° (0,5 % in Chloroform). Die Mutterlauge wird mit Dichlormethan extrahiert, getrocknet und eingedampft. Beide Fraktionen werden zusammen in 6 ml Tetrahydrofuran gelöst und die Lösung in 50 ml kondensierten, wasserfreien Ammoniak eingetropft. Dann versetzt man mit 160 mg Lithium und rührt die blaue Lösung 30 Minuten bei - 40 °C. Nacheinander gibt man festes Ammoniumchlorid bis zur Entfärbung und 5 ml Wasser zu, dampft den Ammoniak ab und verdünnt mit 80 ml Wasser. Nach 30 Minuten Rühren im Eisbad werden die ausgeschiedenen Kristalle abgesaugt und im Vakuum getrocknet, Ausbeute 643 mg (94 % der Theorie), $[\alpha]_D$ = + 3 ° (0,5 % in Chloroform).

Aus dem betreffenden Alkohol werden analog dargestellt:
1,1-Diethyl-3-(2,13-dimethyl-6-propyl-8α-ergolinyl)-harnstoff, Ausbeute 76 %.
1,1-Diethyl-3-(2-ethyl-13-methyl-6-propyl-8α-ergolinyl)-harnstoff, Ausbeute 81 %.

**Beispiel 7**

3-(13-Acetyl-2,6-dimethyl-8α-ergolinyl)-1,1-diethyl-harnstoff

In 70 ml Dichlormethan löst man 2,4 g wasserfreies Aluminiumchlorid und 1,3 ml Acetylchlorid und rührt 15 Minuten bei Raumtemperatur. Dazu gibt man 708 mg 1,1-Diethyl-3-(2,6-dimethyl-8α-ergolinyl)-harnstoff (2 mmol) gelöst in 30 ml Dichlormethan und rührt 30 Minuten bei Raumtemperatur. Man fügt Eis und nach 15 Minuten Rühren eine Lösung von 2,8 g Weinsäure in 80 ml Wasser zu. Nach weiteren 15 Minuten macht man mit konz. Ammoniak alkalisch, trennt die organische Phase ab und extrahiert die Wasserphase. Alle organischen Phasen werden mit Natriumsulfat getrocknet und eingedampft, der Rückstand an Kieselgel mit Dichlormethan/Methanol chromatographiert, Ausbeute 454 mg.

In analoger Weise werden dargestellt:
3-(13-Acetyl-2-methyl-6-propyl-8α-ergolinyl)-1,1-diethyl-harnstoff, Ausbeute 61 %.
3-(13-Acetyl-2-ethyl-6-methyl-8α-ergolinyl)-1,1-diethyl-harnstoff, Ausbeute 38 %.
3-(13-Acetyl-2-ethyl-6-propyl-8α-ergolinyl)-1,1-diethyl-harnstoff, Ausbeute 58 %.
3-(13-Acetyl-2-brom-6-methyl-8α-ergolinyl)-1,1-diethyl-harnstoff, Ausbeute 67 %.
3-(13-Acetyl-2-brom-6-propyl-8α-ergolinyl)-1,1-diethyl-harnstoff, Ausbeute 47 %.
3-(13-Acetyl-2-chlor-6-propyl-8α-ergolinyl)-1,1-diethyl-harnstoff, Ausbeute 71 %.
3-(13-Acetyl-6-methyl-2-methylthio-8α-ergolinyl)-1,1-diethyl-harnstoff, Ausbeute 44 %, $[\alpha]_D$ = + 7 ° (0,5 % in Chloroform).

**Beispiel 8**

1,1-Diethyl-3-[2,6-dimethyl-13-(1-hydroxyethyl)-8α-ernolinyl]-harnstoff

406 mg 3-(13-Acetyl-2,6-dimethyl-8α-ergolinyl)-1,1-diethyl-harnstoff (1 mmol) werden in 50 ml Tetrahydrofuran mit 200 mg Lithiumaluminiumhydrid wie in Beispiel 4 beschrieben reduziert, aufgearbeitet und aus Essigester kristallisiert, Ausbeute 216 mg (53 % der Theorie), $[\alpha]_D$ = + 12 ° (0,1 % in Pyridin).
Analog werden die folgenden Alkohole dargestellt:
1,1-Diethyl-3-[13-(1-hydroxyethyl)-2-methyl-6-propyl-8α-ergolinyl]-harnstoff, Ausbeute 75 %.
1,1-Diethyl-3-[2-ethyl-13-(1-hydroxyethyl)-6-propyl-8α-ergolinyl]-harnstoff, Ausbeute 57 %.
1,1-Diethyl-3-[2-ethyl-13-(1-hydroxyethyl)-6-methyl-8α-ergolinyl]-harnstoff, Ausbeute 43 %.
3-[2-Brom-13-(1-hydroxyethyl)-6-methyl-8α-ergolinyl]-1,1-diethyl-harnstoff, Ausbeute 51 %.
3-[2-Brom-13-(1-hydroxyethyl)-6-propyl-8α-ergolinyl]-1,1-diethyl-harnstoff, Ausbeute 61 %.
3-[2-Chlor-13-(1-hydroxyethyl)-6-propyl-8α-ergolinyl]-1,1-diethyl-harnstoff, Ausbeute 47 %.
1,1-Diethyl-3-[13-(1-hydroxyethyl)-6-methyl-2-methylthio-8α-ergolinyl]-harnstoff, Ausbeute 63 %.

**Beispiel 9**

1,1-Diethyl-3-(13-ethyl-2,6-dimethyl-8α-ergolinyl)-harnstoff

280 mg 1,1-Diethyl-3-[2,6-dimethyl-13-(1-hydroxyethyl)-8α-ergolinyl)-harnstoff (0,7 mmol) werden in 14 ml Essigsäure gelost und mit 700 mg Natriumborhydrid (Tabletten) 15 Minuten bei Raumtemperatur gerührt. Dann gibt man Eis zu, rührt weitere 15 Minuten und macht mit konz. Ammoniak alkalisch. Die Substanz wird mit Dichlormethan extrahiert, die organischen Phasen getrocknet und eingedampft, der Rückstand an Kieselgel mit Dichlormethan/Methanol chromatographiert. Die isolierte Substanz wird aus Essigester/Hexan kristallisiert, Ausbeute 176 mg (65 % der Theorie), $[\alpha]_D$ = + 5 ° (0,5 % in Chloroform).
Analog werden die entsprechenden Alkohole reduziert:
1,1-Diethyl-3-(13-ethyl-2-methyl-6-propyl-8α-ergolinyl)-harnstoff, Ausbeute 73 % der Theorie.
1,1-Diethyl-3-(2,13-diethyl-6-methyl-8α-ergolinyl]-harnstoff, Ausbeute 64 % der Theorie.
1,1-Diethyl-3-(2,13-diethyl-6-propyl-8α-ergolinyl)-harnstoff, Ausbeute 47 % der Theorie.
3-(2-Brom-13-ethyl-6-methyl-8α-ergolinyl)-1,1-diethyl-harnstoff, Ausbeute 28 %.
3-(2-Brom-13-ethyl-6-propyl-8α-ergolinyl)-1,1-diethyl-harnstoff, Ausbeute 23 %.
3-(2-Chlor-13-ethyl-6-propyl-8α-ergolinyl)-1,1-diethyl-harnstoff, Ausbeute 44 %.
1,1-Diethyl-3-(13-ethyl-2-methylthio-6-propyl-8α-ergolinyl)-harnstoff, Ausbeute 33 %.

**Beispiel 10**

3-(2-Brom-6,13-dimethyl-8α-ergolinvl)-1,1-diethyl-harnstoff

Aus 3-(2-Brom-13-hydroxymethyl-6-methyl-8α-ergolinyl)-1,1-diethyl-harnstoff durch Reduktion wie im Beispiel 9 beschrieben, Ausbeute 43 %.
Analog werden synthetisiert:
3-(2-Brom-13-methyl-6-propyl-8α-ergolinyl)-1,1-diethyl-harnstoff, Ausbeute 24 %.
3-(2-Chlor-13-methyl-6-propyl-8α-ergolinyl)-1,1-diethyl-harnstoff, Ausbeute 45 %.
1,1-Diethyl-3-(13-methyl-2-methylthio-6-propyl-8α-ergolinyl)-harnstoff, Ausbeute 37 %.

**Beispiel 11**

1,1-Diethyl-3-[13-(1-hydroxy-1-methyl-ethyl)-2-6-dimethyl-8α-ergolinyl)-harnstoff

396 mg 3-(13-Acetyl-2,6-dimethyl-8α-ergolinyl)-1,1-diethyl-harnstoff (1 mmol) werden in 30 ml trockenem Tetrahydrofuran gelöst und die Lösung auf - 65 °C gekühlt. Es werden 0,8 ml einer 1,6 molaren Lösung von Methyllithium in Ether zugegeben (1,3 mmol), dann läßt man die Lösung auf Raumtemperatur anwärmen und rührt weitere 30 Minuten. Man gießt auf Eis, macht mit konz. Ammoniaklösung alkalisch und extrahiert mit Essigester. Die organischen Phasen werden getrocknet und abgedampft, der Rückstand an Kieselgel mit Dichlormethan/Methanol chromatographiert, Ausbeute 285 mg (67 % der Theorie).

Analog werden aus den 13-Acetylverbindungen dargestellt:

1,1-Diethyl-3-[13-(1-hydroxy-1-methyl-ethyl)-2-methyl-6-propyl-8α-ergolinyl] -harnstoff, Ausbeute 54 %.

3-[2-Brom-13-(1-hydroxy-1-methyl-ethyl)-6-propyl-8α-ergolinyl]-1,1-diethylharnstoff, Ausbeute 73 %.

3-[2-Chlor-13-(1-hydroxy-1-methyl-ethyl)-6-propyl-8α-ergolinyl)-1,1-diethylharnstoff, Ausbeute 67 %.

**Beispiel 12**

1,1-Diethyl-3(2,6-dimethyl-13-isopropenyl-8α-ergolinyl)-harnstoff

Man löst 208 mg 1,1-Diethyl-3-[13-(1-hydroxy-1-methyl-ethyl)-2,6-dimethyl-8α-ergolinyl]-harnstoff (0,5 mmol) in 20 ml wasserfreiem Tetrahydrofuran, versetzt mit 0,7 ml Triethylamin (5 mmol) und 0,4 ml Methansulfonsäurechlorid 15 mmol) und rührt 30 Minuten bei Raumtemperatur. Man gibt Eis zur Mischung, macht mit konz. Ammoniak alkalisch und schüttelt mit Essigester aus. Der Rückstand wird an Kieselgel mit Dichlomethan/Methanol chromatographiert, Ausbeute 99 mg (50 % der Theorie).

Auf analoge Weise werden dargestellt:

1,1-Diethyl-3-(13-isopropenyl-2-methyl-6-propyl-8α-ergolinyl)-harnstoff, Ausbeute 34 %.

3-(2-Brom-13-isopropenyl-6-propyl-8α-ergolinyl)-1,1-diethyl-harnstoff, Ausbeute 45 %.

**Beispiel 13**

1,1-Diethyl-3-(2,6-dimethyl-13-isopropyl-8α-ergolinyl)-harnstoff

Man löst 1,1-Diethyl-3-[13-(1-hydroxy-1-methyl-1-ethyl)-2,6-dimethyl-8α-ergolinyl]-harnstoff (0,5 mmol) in 5 ml Eisessig und gibt 0,25 g Natriumborhydrid zu. Nach 15 Minuten Rühren bei Raumtemperatur gibt man Eis zu, macht mit konz. Ammoniak alkalisch und extrahiert mit Essigester. Die organischen Phasen wrden mit Natriumsulfat getrocknet und eingedampft, der Rückstand an Kieselgel mit Dichlormethan/Methanol chromatographiert, Ausbeute 76 mg (38 % der Theorie).

Auf analoge Weise werden dargestellt:

1,1-Diethyl-3-(13-isopropyl-2-methyl-6-propyl-8α-ergolinyl)-harnstoff, Ausbeute 47 %.

3-(2-Chlor-13-isopropyl-6-propyl-8α-ergolinyl)-1,1-diethyl-harnstoff, Ausbeute 33 %.

**Beispiel 14**

3-(2-Brom-13-chlor-6-methyl-8α-ergolinyl)-1,1-diethyl-harnstoff

In 100 ml Trifluoressigsäure löst man 2,1 g 3-(2-Brom-6-methyl-8α-ergolinyl)-1,1-diethyl-harnstoff (5 mmol) und 388 mg Trichlorisocyanursäure (1,67 mmol) bei Raumtemperatur. Nach 15 Minuten gibt man Eis zu, macht mit konz. Ammoniak alkalisch und schüttelt mit Dichlormethan aus. Die organischen Phasen wrden getrocknet und eingedampft, der Rückstand chromatographiert und das Produkt aus Essigester/Ether kristallisiert, Ausbeute 480 mg (21 % der Theorie).

In analoger Weise werden dargestellt:

3-(2-Brom-13-chlor-6-propyl-8α-ergolinyl)-1,1-diethyl-harnstoff, Ausbeute 38 %.

3-(2,13-Dichlor-6-propyl-8α-ergolinyl)-1,1-diethyl-harnstoff, Ausbeute 33 %.

3-(13-Chlor-2-methyl-6-propyl-8α-ergolinyl)-1,1-diethyl-harnstoff, Ausbeute 21 %.

3-(13-Chlor-2,6-dimethyl-8α-ergolinyl)-1,1-diethyl-harnstoff, Ausbeute 28 %.

3-(13-Chlor-2-ethyl-6-propyl-8α-ergolinyl)-1,1-diethyl-harnstoff, Ausbeute 41 %.

**Beispiel 15**

3-(2-Brom-13-iod-6-methyl-8α-ergolinyl)-1,1-diethyl-harnstoff

42 mg 3-(2-Brom-6-methyl-8α-ergolinyl)-1,1-diethyl-harnstoff (0,1 mmol) werden in 2 ml Trifluoressigsäure gelöst und mit 22 mg N-Iodsuccinimid (0,1 mmol) versetzt. Nach 15 Minuten Rühren bei Raumtemperatur gibt man Eis zu, macht mit Ammoniak alkalisch und schüttelt mit Dichlormethan aus. Die organischen Phasen werden getrocknet und eingedampft, der Rückstand chromatographiert. Die reine Substanz kristallisiert aus Dichlormethan, Ausbeute 9 mg (16 % der Theorie).

In analoger Weise werden dargestellt:
3-(2-Brom-13-iod-6-propyl-8α-ergolinyl)-1,1-diethyl-harnstoff, Ausbeute 25 %.
1,1-Diethyl-3-(2,6-dimethyl-13-iod-8α-ergolinyl)-harnstoff, Ausbeute 17 %.
1,1-Diethyl-3-(13-iod-2-methyl-6-propyl-8α-ergolinyl)-harnstoff, Ausbeute 29 %.

**Beispiel 16**

1,1-Diethyl-3-(2,6-dimethyl-13-methylthio-8α-ergolinyl)-harnstoff

Man löst 3,54 g 1,1-Diethyl-3-(2,6-dimethyl-8α-ergolinyl)-harnstoff (10 mmol) in 200 ml Trifluoressigsäure und versetzt im Abstand von 15 Minuten in drei Portionen mit je 0,98 g Dimethyl-methylthio-sulfonium-tetrafluorborat (15 mmol) bei Raumtemperatur. Nach 15 Minuten gießt man die Mischung auf Eis, macht Ammoniak alkalisch und schüttelt mit Dichlormethan aus. Die organischen Phasen werden mit Natriumsulfat getrocknet und eingedampft, der Rückstand an Kieselgel mit Dichlormethan/Methanol/Hexan chromatographiert. Die Substanz wird aus Essigester/Hexan kristallisiert, Ausbeute 138 mg (4 % der Theorie).

In analoger Weise wird dargestellt:
1,1-Diethyl-3-(2-methyl-13-methylthio-6-propyl-8α-ergolinyl)-harnstoff, Ausbeute 13 %.

**Beispiel 17**

1,1-Diethyl-3-(2,6,13-trimethyl-8α-ergolinyl)-thioharnstoff

In 20 ml Dichlormethan löst man 0,5 ml frisch destilliertes Phosphoroxychlorid (5,6 mmol) und 368 mg 1,1-Diethyl-3-(2,6-13-trimethyl-8α-ergolinyl)-harnstoff (1 mmol) bei - 20 °C und läßt die Mischung über Nacht bei Raumtemperatur rühren. Nun werden die flüchtigen Anteile im Vakuum abgezogen, der Rückstand in 40 ml Acetonitril gelöst und mit einer Lösung von 0,8 g Kaliumxanthogenat (5,6 mmol) in 80 ml Acetonitril versetzt. Man rührt 2 Stunden bei Raumtemperatur, gibt dann Eis und konz. Ammoniaklösung zu und schüttelt mit Dichlormethan aus. Die organischen Phasen werden mit Natriumsulfat getrocknet und eingedampft, der Rückstand an Kieselgel mit Essigester chromatographiert und aus Essigester/Diisopropylether kristallisiert, Ausbeute 43 %.

In analoger Weise werden die folgenden Thioharnstoffe durch Thiolierung der Harnstoffe dargestellt:
1,1-Diethyl-3-(2,13-dimethyl-6-propyl-8α-ergolinyl)-thioharnstoff, Ausbeute 42 %.
1,1-Diethyl-3-(13-ethyl-2-methyl-6-propyl-8α-ergolinyl)-thioharnstoff, Ausbeute 56 %.
3-(13-Chlor-2-methyl-6-propyl-8α-ergolinyl)-1,1-diethyl-thioharnstoff, Ausbeute 37 %.
1,1-Diethyl-3-(2-ethyl-13-methyl-6-propyl-8α-ergolinyl)-thioharnstoff
3-(2-Brom-6,13-dimethyl-8α-ergolinyl)-1,1-diethyl-thioharnstoff, Ausbeute 61 %.
3-(2-Brom-13-methyl-6-propyl-8α-ergolinyl)-1,1-diethyl-thioharnstoff, Ausbeute 34 %.
3-(2-Brom-13-iod-6-propyl-8α-ergolinyl)-1,1-diethyl-thioharnstoff, Ausbeute 23 %.
3-(2-Brom-13-chlor-6-propyl-8α-ergolinyl)-1,1-diethyl-thioharnstoff, Ausbeute 59 %.
3-(2-Chlor-13-methyl-6-propyl-8α-ergolinyl)-diethyl-thioharnstoff, Ausbeute 65 %.
3-(2,13-Dichlor-6-propyl-8α-ergolinyl)-1,1-diethyl-thioharnstoff, Ausbeute 42 %.
1,1-Diethyl-3-(13-methyl-2-methylthio-6-propyl-8α-ergolinyl)-thioharnstoff, Ausbeute 34 %.

## Patentansprüche

**1.** Verbindungen der Formel I und deren Säureadditionssalze

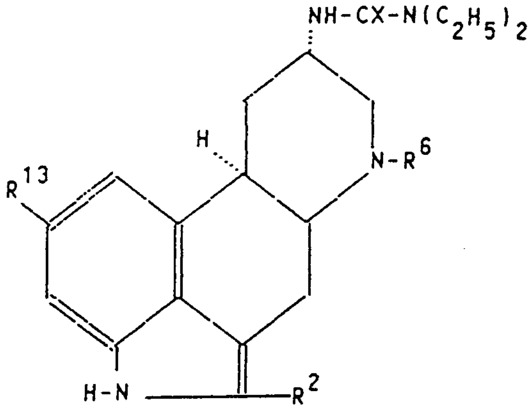

I

worin

- $R^2$ Halogen, $C_{1-6}$-Alkyl oder -S-$C_{1-4}$-Alkyl,
- $R^6$ $C_{1-6}$-Alkyl, $C_{3-6}$-Alkenyl oder $C_{3-5}$-Cycloalkyl-$C_{1-2}$-alkyl,
- X Sauerstoff oder Schwefel,
- $R^{13}$ Chlor, Jod, -S-$C_{1-4}$-Alkyl, $C_{1-6}$-Alkyl, $C_{2-6}$-Alkenyl, 1,3-Dithiolan-2-yl, -CO-H oder -$CR^4R^5OH$ ist und $R^4$ und $R^5$ jeweils Wasserstoff oder $C_{1-5}$-Alkyl bedeuten.

**2.** 1,1-Diethyl-3-(2,6,13-trimethyl-8α-ergolinyl]-harnstoff
1,1-Diethyl-3-(2,13-dimethyl-6-propyl-8α-ergolinyl)-harnstoff
1,1-Diethyl-3-(13-ethyl-2,6-dimethyl-8α-ergolinyl)-harnstoff
1,1-Diethyl-3-(13-ethyl-2-methyl-6-propyl-8α-ergolinyl)-harnstoff
1,1-Diethyl-3-(2,6-dimethyl-13-isopropyl-8α-ergolinyl)-harnstoff
8α-(3,3-Diethylureido)-2-methyl-6-propyl-ergolin-13-carbaldehyd
3-(2-Brom-13-chlor-6-methyl-8α-ergolinyl)-1,1-diethyl-harnstoff
3-(2-Brom-)-13-chlor-6-propyl-8α-ergolinyl)-1,1-diethyl-harnstoff
1,1-Diethyl-3-(2,6-dimethyl-13-methylthio-8α-ergolinyl)-harnstoff
1,1-Diethyl-3-(2,13-dimethyl-6-propyl-8α-ergolinyl)-thioharnstoff nach Anspruch I.

**3.** Arzneimittel auf Basis der Verbindungen nach Anspruch 1 und 2.

**4.** Verfahren zur Herstellung der Verbindungen nach Anspruch 1 dadurch gekennzeichnet, daß man
a) Verbindungen der Formel II

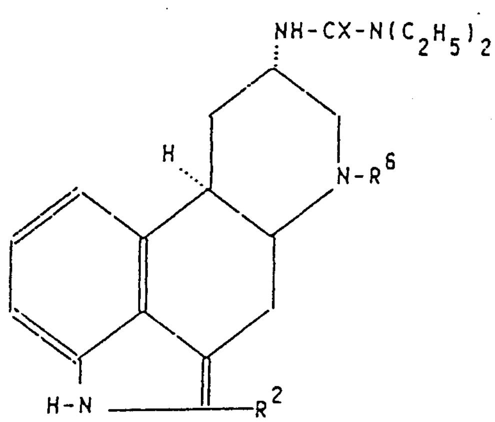

II

worin $R^2$, $R^6$ und X die obige Bedeutung haben, in Gegenwart einer Säure mit einem elektrophilen Agens umsetzt oder

b) Verbindungen der Formel III

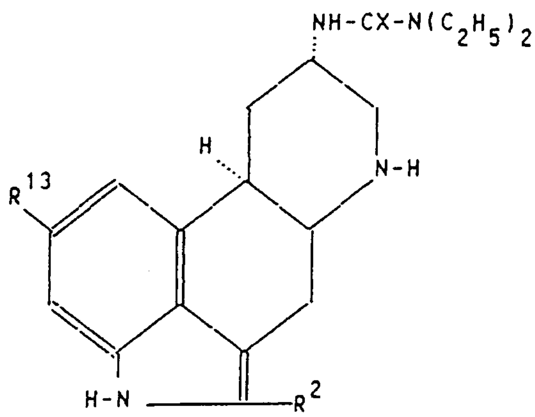

III

worin $R^2$, $R^{13}$ und X die obige Bedeutung haben, alkyliert oder alkenyliert zu Verbindungen mit $R^6$ in der obigen Bedeutung

und gewünschtenfalls anschließend

α) Verbindungen mit $R^{13}$ = -CO-$R^3$ mit $R^3$ = H oder $C_{1-5}$-Alkyl reduziert zu Verbindungen mit $R^{13}$ = -$CR^4R^5OH$ und diese gewünschtenfalls dehydratisiert zu Verbindungen mit $R^{13}$ = $C_{2-6}$-Alkenyl oder reduziert zu Verbindungen mit $R^{13}$ = $C_{1-6}$-Alkyl oder

β) Verbindungen mit $R^{13}$ = 1,3-Dithiolan-2-yl überfuhrt in Verbindungen mit $R^{13}$ = -CHO oder $CH_3$ oder

γ) den Harnstoff in den Thioharnstoff überführt oder

δ) die Isomeren trennt oder die Säureadditionssalze bildet.

**5.** Verbindungen der Formel IV

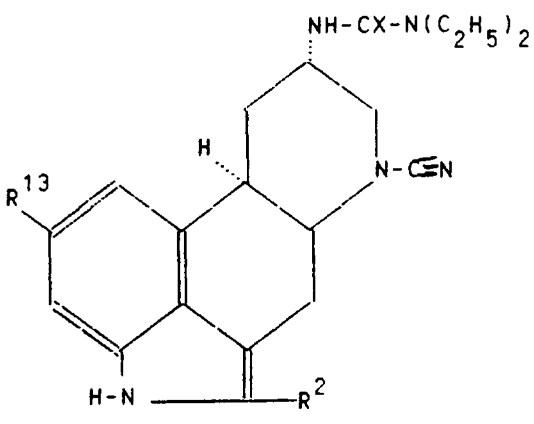

IV

worin

$R^2$, $R^{13}$ und X die obige Bedeutung haben.

## Claims

1. Compounds of the formula I and acid addition salts thereof

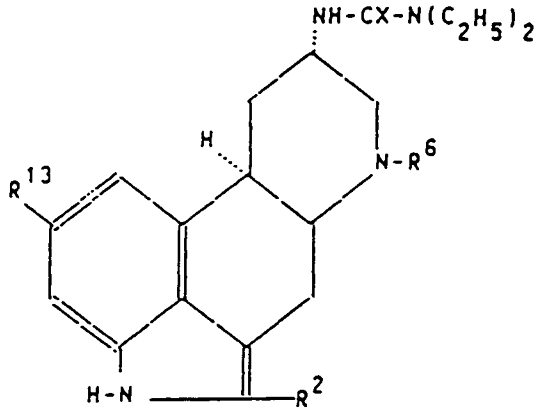

(I),

wherein

$R^2$ is halogen, $C_{1-6}$-alkyl or -S-$C_{1-4}$-alkyl,

$R^6$ is $C_{1-6}$-alkyl, $C_{3-6}$-alkenyl or $C_{3-5}$-cycloalkyl-$C_{1-2}$-alkyl,

X is oxygen or sulfur,

$R^{13}$ is chlorine, iodine, -S-$C_{1-4}$-alkyl, $C_{1-6}$-alkyl, $C_{2-6}$-alkenyl, 1,3-dithiolan-2-yl, -CO-H or -$CR^4R^5OH$, and $R^4$ and $R^5$ each represents hydrogen or $C_{1-5}$-alkyl.

2. 1,1-Diethyl-3-(2,6,13-trimethyl-8α-ergolinyl)-urea
1,1-Diethyl-3-(2,13-dimethyl-6-propyl-8α-ergolinyl)-urea
1,1-Diethyl-3-(13-ethyl-2,6-dimethyl-8α-ergolinyl)-urea
1,1-Diethyl-3-(13-ethyl-2-methyl-6-propyl-8α-ergolinyl)-urea
1,1-Diethyl-3-(2,2-dimethyl-13-isopropyl-8α-ergolinyl)-urea
8α-(3,3-Diethylureido)-2-methyl-6-propyl-ergoline-13-carbaldehyde
3-(2-bromo-13-chloro-6-methyl-8α-ergolinyl)-1,1-diethyl-urea
3-(2-bromo-13-chloro-6-propyl-8α-ergolinyl)-1,1-diethyl-urea
1,1-Diethyl-3-(2,6-dimethyl-13-methylthio-8α-ergolinyl)-urea
1,1-Diethyl-3-(2,13-dimethyl-6-propyl-8α-ergolinyl)-urea according to claim 1.

3. Medicaments based on compounds according to claims 1 and 2.

4. Process for the manufacture of the compounds according to claim 1, characterised in that
a) compounds of the formula II

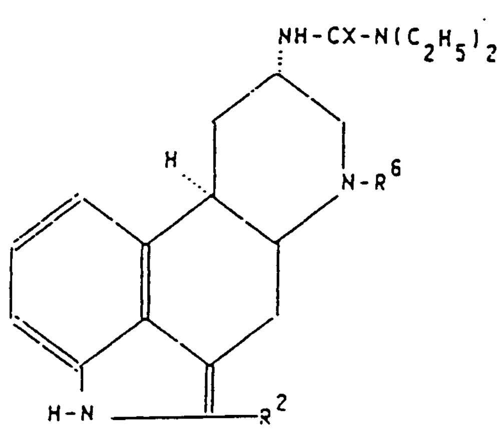

(II),

wherein $R^2$, $R^6$ and X have the meanings given above, are reacted in the presence of an acid with an electrophilic agent or

b) compounds of the formula III

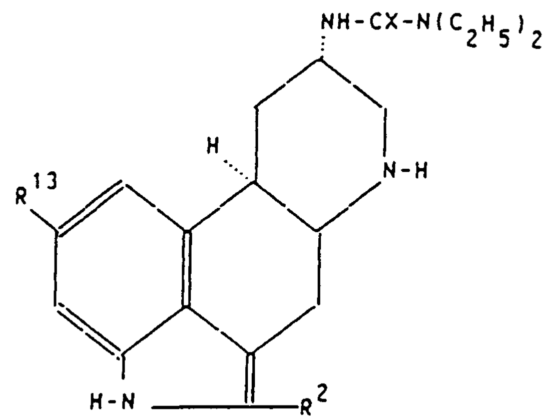

III,

wherein $R^2$, $R^{13}$ and X have the meanings given above, are alkylated or alkenylated to form compounds wherein $R^6$ has the meanings given above

and, if desired, subsequently

α) compounds wherein $R^{13}$ = -CO-$R^3$, wherein $R^3$ = H or $C_{1-5}$-alkyl, are reduced to compounds wherein $R^{13}$ = -$CR^4R^5OH$ and, if desired, the latter are dehydrated to form compounds wherein $R^{13}$ = $C_{2-6}$-alkenyl or reduced to compounds wherein $R^{13}$ = $C_{1-6}$-alkyl, or

β) compounds wherein $R^{13}$ = 1,3-dithiolan-2-yl are converted into compounds wherein $R^{13}$ = -CHO or $CH_3$, or

γ) the urea is converted into the thiourea, or

δ) the isomers are separated or the acid addition salts are formed.

**5.** Compounds of the formula IV

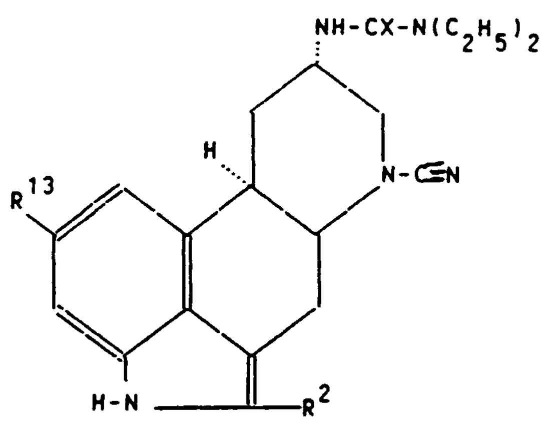

IV,

wherein

$R^2$, $R^{13}$ and X have the meanings given above.

## Revendications

**1.** Composés de formule I et leurs sels obtenus par l'addition d'acide

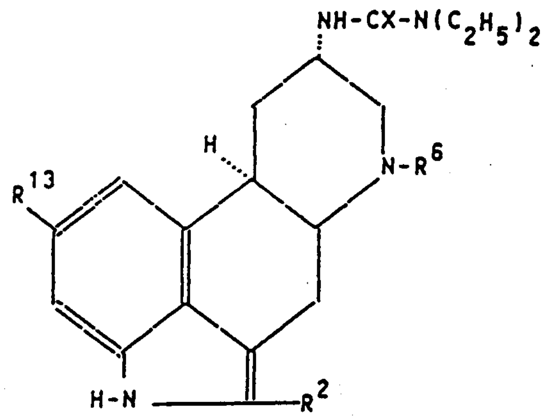

I

dans laquelle

- $R^2$ est un halogène, un alkyle en $C_{1-6}$ ou un -S-(alkyle en $C_{1-4}$),
- $R^6$ est un alkyle en $C_{1-6}$, alcényle en $C_{3-6}$ ou (cycloalkyle en $C_{3-5}$)-(alkyle en $C_{1-2}$),
- X est un oxygène ou un soufre,
- $R^{13}$ est un chlore, un iode, un -S-(alkyle en $C_{1-4}$), un alkyle en $C_{1-6}$, un alcényle en $C_{2-6}$, un 1,3-dithiolane-2-yle, un -CO-H ou un -$CR^4R^5$OH et $R^4$ et $R^5$ signifient chaque fois un hydrogène ou un alkyle en $C_{1-5}$.

**2.** 1,1-Diéthyl-3-(2,6,13-triméthyl-8α-ergolinyl]-urée,
1,1-diéthyl-3-(2,13-diméthyl-6-propyl-8α-ergolinyl)-urée,
1,1-diéthyl-3-(13-éthyl-2,6-diméthyl-8α-ergolinyl)-urée,
1,1-diéthyl-3-(13-éthyl-2-méthyl-6-propyl-8α-ergolinyl)-urée,
1,1-diéthyl-3-(2,6-diméthyl-13-isopropyl-8α-ergolinyl)-urée,
8α-(3,3-diéthyluréido)-2-mèthyl-6-propyl-ergolin-13-carbaldéhyde,
3-(2-bromo-13-chloro-6-méthyl-8α-ergolinyl)-1,1-diéthylurée,
3-(2-bromo-13-chloro-6-propyl-8α-ergolinyl)-1,1-diéthylurée,
1,1-diéthyl-3-(2,6-diméthyl-13-méthylthio-8α-ergolinyl)-urée,
1,1-diéthyl-3-(2,13-diméthyl-6-propyl-8α-ergolinyl)-thiourée,
selon la revendication 1.

**3.** Médicaments à base des composés selon la revendication 1 ou 2.

**4.** Procédé de préparation des composés selon la revendication 1 caractérisé en ce que
a) on transforme les composés de formule II

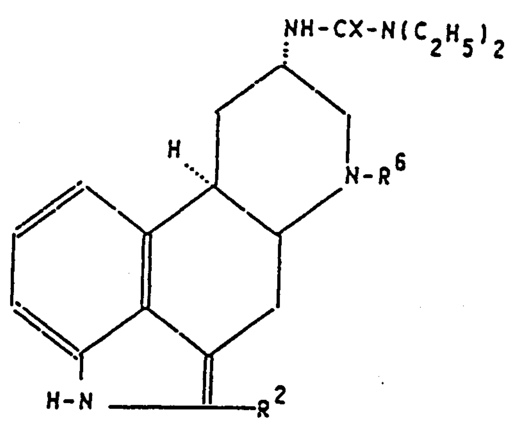

II

dans laquelle $R^2$, $R^6$ et X ont les significations ci-dessus, en présence d'un acide avec un agent électrophile ou

b) on alkyle ou on alcényle sur des composés de formule III

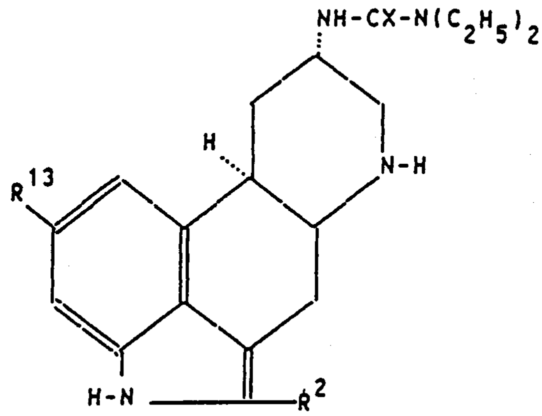

III

dans laquelle $R^2$, $R^{13}$ et X ont les significations ci-dessus, conduisant aux composés avec $R^6$ ayant la signification ci-dessus

et, ensuite, suivant le cas désiré,

α) on réduit les composés ayant $R^{13}$ = -CO-$R^3$ avec $R^3$ = H ou alkyle en $C_{1-5}$ pour donner les composés ayant $R^{13}$ = -$CR^4R^5$OH et selon le cas désiré on déshydrate ces composés pour donner les composés ayant $R^{13}$ = alcényle en $C_{2-6}$ ou on réduit en composés ayant $R^{13}$ = alkyle en $C_{1-6}$ ou

β) on convertit les composés ayant $R^{13}$ = 1,3-dithiolane-2-yle en composés ayant $R^{13}$ = -CHO ou -$CH_3$ ou

γ) on convertit l'urée en thiourée ou

δ) on sépare les isomères ou on forme les sels d'acides.

**5.** Composé de formule IV

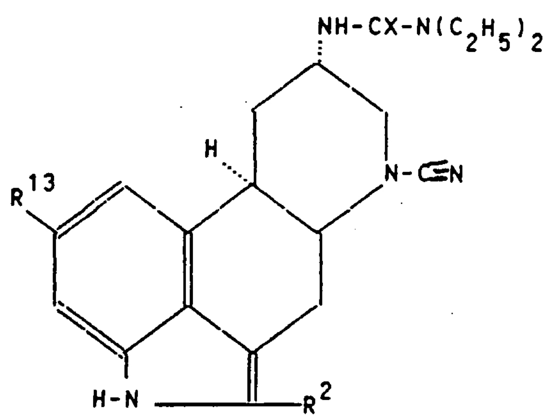

IV

dans laquelle
$R^2$, $R^{13}$ et X ont la signification ci-dessus.